# FASCICULE DE BREVET EUROPEEN

(11) **EP 1 338 270 B1**
(45) Date de publication et mention de la délivrance du brevet: **21.01.2009**
(21) Numéro de dépôt: 02290833.9
(22) Date de dépôt: 04.04.2002
(51) Int. Cl.: A61K 8/60, A61K 36/73, A61K 8/97, A61K 31/05, A61K 31/70, A23L 1/30, A61Q 19/00

(54) **Utilisation dans un traitement cosmétique d'une fraction phénolique riche en dihydrochalcones**
Verwendung einer dihydrochalcon-reichen phenolischen Fraktion für eine kosmetische Behandlung
Use of a dihydrochalcone-rich phenolic fraction in a cosmetic composition

(30) Priorité: 26.02.2002 FR 0202418
(43) Date de publication de la demande: 27.08.2003
(73) Titulaire: Diana Ingrédients, 56250 Saint-Nolff (FR)
(72) Inventeur: Gaudout, David, 35300 Fougeres (FR); Megard, Denis, 35460 Saint Brice en Cogles (FR); Lejard, Frédéric, 56610 Arradon (FR)
(74) Mandataire: Dossmann, Gérard

(56) Documents cités:
- EP-A- 0 657 169
- EP-A- 0 781 544
- EP-A- 0 797 427
- EP-A- 1 243 586
- WO-A-01/15706
- WO-A-01/24806
- WO-A-02/34073
- US-A- 4 760 135
- PAGANGA G ET AL: "THE POLYPHENOLIC CONTENT OF FRUIT AND VEGETABLES AND THEIR ANTIOXIDANT ACTIVITIES. WHAT DOES A SERVING CONSTITUTE?" FREE RADICAL RESEARCH,, YVERDON, CH, vol. 30, no. 2, 1999, pages 153-162, XP000918290 ISSN: 1071-5762
- DATABASE CA [Online] CHEMICAL ABSTRACTS SERVICE, COLUMBUS, OHIO, US; XP002095439 extrait de STN Database accession no. 122:131234

## Description

La présente invention est relative à l'utilisation d'une fraction polyphénolique riche en dihydrochalcones dans le traitement cosmétique de mammifères afin de limiter la charge pondérale, améliorer l'aspect esthétique du corps et traiter certaines formes d'obésité non pathologiques. L'invention est aussi relative à l'utilisation d'une composition alimentaire ou nutraceutique à base de cette fraction phénolique.

Il est connu que les composés polyphénoliques sont très répandus dans le règne végétal. Les plus abondants sont l'acide chlorogénique, les procyanidines B1 et B2, l'épicatéchine, la phlorétine, la phloridzine et l'acide p-coumarique. De ce fait, de nombreux produits riches en polyphénols existent sur le marché, les plus courants étant extraits du thé vert, des pépins de raisin et de l'écorce de pin (US A 4 698 360, EP A 348 781, EP A 283 349, FR A 1 427 100, FR A 2 092 743, FR A 2 643 073 et FR A 2 372 823).

Il a déjà été décrit dans le brevet EP A 0 657 169 l'extraction d'une fraction polyphénolique à partir de fruits non matures (pesant de 3 à 10 grammes) de la famille des *Rosaceae* (pomme, poire...). La fraction polyphénolique ainsi définie se caractérise par une haute teneur en dérivés de la famille des acides hydroxycinnamiques (acide chlorogénique, cafféique et p-coumarique) et en molécules de la famille des flavanols (catéchine, épicatéchine et procyanidine). La phloridzine de ces extraits issus de fruits immatures représente moins de 7 % en poids de l'ensemble des composés phénoliques et les dihydrochalcones (phloridzine et phlorétine) typiques des rosaceae moins de 9 %. Parmi les composés phénoliques présents dans le règne végétal, la phlorétine et son dérivé glycosylé, la phloridzine sont typiques de la pomme et des autres fruits de la famille des Rosaceae. On trouve la phloridzine en quantité importante dans les pépins et dans l'écorce des arbres, et en quantité bien moindre dans le jus et la peau de pomme.

La phloridzine est connue depuis longtemps pour son activité de blocage de l'assimilation du glucose. Un des mécanismes d'action décrit serait que la phloridzine entre en compétition avec les sucres simples et, par conséquent, limite leur transport dans le sang (Alvarado et Crane, Biochim. Biophys. Acta 56, pp170, 1962). Un autre mécanisme, peut-être lié au précédent, ferait intervenir le blocage des systèmes de transport sodium-dépendants des oses tels que glucose, galactose, xylose... au niveau de l'intestin grêle (Esaki et al., Agric. Biol. Chem. 55, 11, pp2855, 1991. Il semblerait que le transporteur 'naturel' du sucre dispose de deux sites indépendants, le site à affinité 'sucre' et le site à affinité 'phénol', et que la phloridzine, en se liant fortement avec le transporteur par interaction sur les deux sites, bloque le transport des sucres à travers les membranes.

Ces hypothèses ont été confirmées par des travaux *in vivo.* La phloridzine a été étudiée expérimentalement depuis longtemps avec succès pour diminuer la disponibilité du glucose sanguin et provoquer une glycosurie (présence de glucose dans les urines) chez le mouton (Goetsch et Pritchard, . Am. J. Vet. Res. 19, pp637, 1958), la chèvre (Schultz et al., J. Dairy Sci. 32, pp817, 1949) et le bovin (Lyle et al. J. Dairy Sci. 67, pp2255, 1984 ; Young et al. J. Dairy Sci. 57, pp689, 1974. 2 à 4 grammes de phloridzine par jour pendant 48 h en injection sous-cutanée à une vache sont suffisants pour entraîner une diminution drastique de la teneur en glucose et en insuline du plasma sanguin et l'excrétion de 225 à 337 grammes de glucose par jour dans les urines de l'animal traité (Amaral-Phillips et al., J. Dairy Sci. 76, pp752, 1993).

Ce mécanisme de blocage du transport membranaire du glucose est très intéressant en particulier dans les régimes occidentaux pour la prévention du diabète et pour le traitement de certaines formes d'obésité. Deux brevets font ainsi intervenir la phloridzine dans des compositions médicamenteuses destinées à bloquer le transport du glucose. Le brevet CZ 1993000931986 (Valovic) décrit un mélange à base d'acides phosphorique, sulfurique, lactique, de créosote, de trioxyde d'arsenic, de sulfate de sodium et d'extraits de plantes parmi lesquels la phloridzine extraite d'écorce d'arbre fruitier. Le brevet US 5 985 850 (Falk et Asculai) décrit des compositions pharmaceutiques faisant intervenir l'acide hyaluronique comme transporteur de molécules actives (dont la phloridzine ou des molécules de la même famille) pour bloquer le transport du glucose dans certains types de cellules.

Dans le broyat ou jus de pomme, les dihydrochalcones (phlorétine et phloridzine) sont présentes en quantité mineure par rapport aux autres polyphénols. L'acide chlorogénique et les procyanidines sont les polyphénols majoritaires des pommes, que ce soit des pommes 'à cidre' ou 'à couteau', la phloridzine et la phlorétine ne représentant jamais plus de 5 % en poids des polyphénols totaux des pommes mûres (Karadeniz et Ekski, 1996, Sanoner et al., 1999a et b)

Dans les extraits polyphénoliques connus, les proportions entre les différentes molécules phénoliques sont conservées par rapport aux proportions présentes dans les différentes matières premières. On obtient donc classiquement des extraits polyphénoliques riches en acide hydroxycinnamiques (acides cafféique, chlorogénique et coumarique) et pauvres en dihydrochalcones (phloridzine et phlorétine) :

| Composé phénolique | Pomme * En mg / L de jus ou kg de broyat | Extrait polyphénolique connu de pomme En mg / g de poudre |
|---|---|---|
| Acide cafféique | ε (non quantifiable) | 21.7 |
| Catéchine | ε à 150 | 15.1 |
| Acide chlorogénique | 60 à 1200 | 161.0 |
| Procyanidines | 500 à 5000 | 69.6 (B1 et B2) |
| Acide p-coumarique | 1 à 150 | 9.3 |
| Epicatéchine | ε à 1400 | 41.4 |
| phloridzine | 6 à 100 | 32.7 |
| Quercitrine | ε | 1.9 |
| Phlorétine | 5 à 100 | 9.5 |
| Polyphénols totaux (exprimés en équivalent phloridzine) | | 483.4 |

| | | |
|---|---|---|
| ε = quantité non quantifiable * valeurs compilées de mesures sur 15 variétés de pommes à cidre et 3 variétés de pommes à couteau sur 3 campagnes (Karadeniz et Ekski, 1996). Karadeniz F. and Ekski A. 1996. Phenolic compounds in apple juice from différent varieties. Scientific technical Com. Int. Fed. Fruit Juice Producers, 24, pp265-275. Sanoner P., Guyot S., Marnet N. et Drilleau J.F. 1999. Polyphenolic profiles of French cider apples varieties. Dans 'Polyphenols, wines and health', symposium, Bordeaux, 14-16 avril. Sanoner P., Guyot S., Marnet N., Molle D. and Drilleau J.F. 1999. Polyphenol profiles of French cider apple varieties. J. Agric. Food Chem. 47, pp4847-4853. | | |

L'acide cafféique est en fait un produit de dégradation de l'acide chlorogénique car il n'y a que très peu, voire pas du tout, d'acide cafféique naturellement présent dans les pommes (Fiedler, 1954, Arzneimittel-Forsch 4, 41).

La demanderesse s'est intéressée à une fraction phénolique de la pomme, cette dernière étant bien connue pour ses effets bénéfiques sur la santé (Blackholly H. Nut. and Food Sci., 109, pp2-4, 1987). La demanderesse a mis au point l'utilisation cosmétique de fractions phénoliques riches en dihydrochalcones, qui constitue l'objet de l'invention.

Un autre objet est constitué par les applications de cette fraction comme complément alimentaire, nutraceutique ou cosmétique dans le cadre d'un traitement cosmétique visant à améliorer l'aspect esthétique du corps.

D'autres objets apparaîtront à la lecture de la description et des exemples qui suivent.

La présente invention a pour objet, dans le traitement cosmétique de mammifères, l'utilisation de fractions phénoliques riches en dihydrochalcones à partir de fruits de la famille des *Rosaceae.* Ces fractions phénoliques riches en dihydrochalcones présentent d'excellentes propriétés de régulation du métabolisme glucidique, plus précisément la propriété de réduire l'assimilation des oses, notamment du glucose, par blocage des systèmes de transport des oses. Elles jouent, dans le cadre d'un traitement cosmétique visant à améliorer l'aspect esthétique du corps, un rôle actif dans la limitation de la charge pondérale, le contrôle de la prise de poids et dans certaines formes d'obésité non pathologiques.

L'utilisation de ces fractions dans les préparations alimentaires ou nutraceutiques pour la limitation de la charge pondérale, le contrôle de la prise de poids et certaines formes d'obésité non pathologiques constitue l'objet de l'invention.

Les fractions polyphénoliques riches en dihydrochalcones utilisées dans l'invention comprennent au moins 10 % en poids de polyphénols et de préférence 50 %, dont 10 % au moins en poids est composé de phloridzine et de préférence entre 10 et 70 %. Ces extraits peuvent contenir également de l'acide chlorogénique, de l'épicatéchine, des procyanidines, de la quercitrine, de l'acide p-coumarique, ainsi que de la phlorétine.

Une composition particulièrement préférée de ces fractions polyphénoliques est qu'elles contiennent en poids :
- plus de 10 % de polyphénols totaux, préférentiellement plus de 50 %,
- au moins 30 % des polyphénols en phloridzine, et de préférence de 30 à 40 % en poids,
- au plus 11 % des polyphénols en acide chlorogénique, et de préférence entre 2 et 11 %,
- au plus 4 % des polyphénols en épicatéchine,
- au plus 2 % des polyphénols en procyanidine B2,
- au plus 2 % des polyphénols en quercitrine,
- au plus 2 % des polyphénols en quercétine,
- au plus 0.5 % des polyphénols en acide p-coumarique, et
- moins de 0.5 % des polyphénols en acide cafféique.

Les fractions polyphénoliques sont caractérisées par le fait que les dihydrochalcones sont présentes dans des proportions supérieures ou égales à 40 % en poids par rapport aux acides hydroxycinnamiques.

L'acide cafféique est présent dans des proportions en poids inférieures à 20 % du poids de phloridzine présent. De préférence, l'acide cafféique représente moins de 1 % en poids des polyphénols totaux des extraits.

La proportion de phloridzine est 9 fois supérieure en poids à celle de la catéchine.

La quantité de phloridzine présente est au moins équivalente en poids à celle de l'acide chlorogénique.

Les fractions polyphénoliques utilisables pour l'application visée peuvent contenir de la phlorétine : par une hydrolyse acide ménagée (Merck Index, 12° édition), on peut transformer la quasi-totalité de la phloridzine en phlorétine.

Dans les fractions phénoliques selon l'invention, les dihydrochalcones sont présentes dans des proportions supérieures ou égales à 40 % en poids par rapport aux acides hydroxycinnamiques.

Les fractions polyphénoliques riches en dihydrochalcones utilisées dans l'invention sont susceptibles d'être obtenues par le procédé d'extraction suivant qui permet l'extraction sélective d'une fraction polyphénolique riche en dihydrochalcones à partir de pommes mûres :
* Les pommes broyées font l'objet d'une ou plusieurs extractions solide / liquide, en présence ou non d'eau ajoutée.
* L'extrait solide humide obtenu est ensuite soit séché, soit liquéfié enzymatiquement pour obtenir un extrait liquide.
* L'extrait solide sec subit de nouvelles extractions pendant un temps compris entre 10 minutes et 2 heures par un solvant organique polaire, de préférence un alcool aliphatique en C1-C4, pur ou en mélange avec de l'eau pour obtenir un extrait organique.
* Celui-ci est évaporé à sec à une température inférieure ou égale à 60 °C de préférence sous pression réduite.
* Ce résidu est ensuite repris par l'eau avant d'être épuisée plusieurs fois, de préférence 4 fois, par un solvant non miscible à l'eau, de préférence l'acétate d'éthyle ou l'acétate de méthyle ou de propyle.
* Les solutions organiques obtenues sont mélangées et évaporées à sec à une température inférieure à 60 °C, préférentiellement inférieure à 50 °C, pour obtenir la fraction polyphénolique qui fait l'objet de la présente invention.
* Par une autre voie, l'extrait solide humide est mélangé à de l'eau en présence d'un mélange enzymatique pendant un temps compris entre 1 et 4 heures à une température comprise entre 30 et 50°C, préférentiellement entre 40 et 45°C, pour obtenir un extrait liquide.
* Cet extrait liquide est clarifié par centrifugation ou par filtration puis par ultrafiltration.
* L'extrait est chargé sur une colonne chromatographique remplie d'une résine adsorbante de type styrène-divinylbenzène. La résine est lavée avec de l'eau acidifiée pour éliminer les impuretés et les sucres résiduels. Les polyphénols sont ensuite élués par une solution hydro-alcoolique renfermant entre 40 et 70 % d'éthanol préférentiellement entre 50 et 60 % en poids. D'autres alcools aliphatiques en C1-C4 peuvent être utilisés tels que le méthanol ou le butanol.
* Si nécessaire, une étape de dé-cirage est introduite en cours de procédé.
* Le produit obtenu par extraction est repris une dernière fois à l'eau avant d'être séché de préférence par atomisation ou lyophilisation pour obtenir une poudre beige contenant au moins 20 % en poids de polyphénols, préférentiellement plus de 50 % de polyphénols, et dont 10 % en poids et préférentiellement entre 10 et 70 % des polyphénols sont des dihydrochalcones, préférentiellement de la phloridzine.

Les fractions sont obtenues par le procédé précédemment décrit de préférence à partir de pommes matures de la famille des rosaceae et en particulier de l'espèce Malus sylvestris Mill.

Cet extrait de pommes comestibles possédant les caractéristiques énoncées, obtenu selon le procédé précédemment décrit, est utilisable en tant que complément alimentaire ou nutraceutique.

L'invention a également pour objet l'utilisation d'une composition cosmétique comprenant, entre autres, la fraction polyphénolique riche en dihydrochalcones précédemment décrite.

L'invention a également pour objet l'utilisation d'une composition alimentaire pour le traitement cosmétique afin de limiter la charge pondérale, pour le traitement cosmétique de certaines formes d'obésité non pathologiques et améliorer l'aspect esthétique du corps.

L'extrait polyphénolique, en diminuant de façon importante l'absorption des sucres par les tissus, a un rôle bénéfique sur le contrôle de la surcharge pondérale et de l'obésité, et peut donc être avantageusement utilisé dans des produits nutraceutiques ou alimentaires à vocation 'minceur'.

Selon le mode d'administration, la composition selon l'invention peut se présenter sous toutes les formes habituellement utilisées en cosmétique telles que par exemple produits alimentaires complémentés, gélules ou boissons.

Les compositions nutraceutiques, alimentaires ou cosmétiques de la présente invention sont formulées classiquement selon l'application à laquelle elle est destinée.

Les exemples suivants illustrent l'invention sans la limiter aucunement.

### Exemple 1 : activité d'inhibition du transport du glucose par la fraction polyphénolique riche en phloridzine

Les transporteurs membranaires du glucose (GLUTs) sont des transporteurs universels du glucose et de l'acide déhydroascorbique (ADHA) des cellules des mammifères et sont essentiels au métabolisme des sucres.

La mesure de l'efficacité de l'extrait polyphénolique à bloquer un transporteur du sucre pour inhiber l'absorption à la fois du glucose et de l'ADHA utilise une méthode décrite par Park et Levine (Park J.B. and Levine M. 2000. J. Nutr. 130, pp1297-1302). Cette méthode fait appel au transporteur GLUT1, existant dans la plupart des tissus. L'activation de ce transporteur permet le transport du glucose depuis le sang jusque dans la cellule.

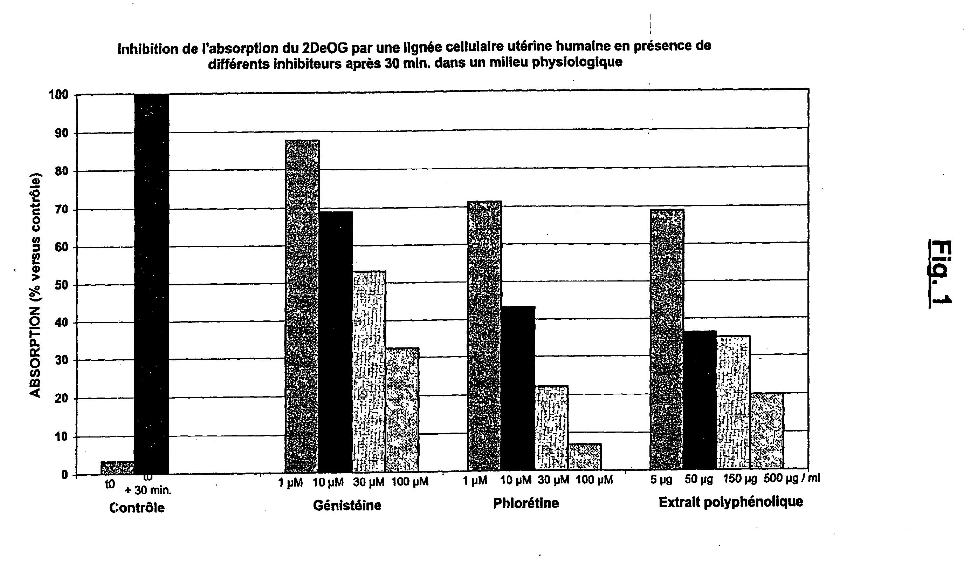

Nous utilisons une lignée cellulaire utérine humaine (Ishikawa Var 1) pour étudier l'effet de l'extrait polyphénolique à des concentrations comprises entre 5 et 500 µg / ml sur la réponse à l'absorption du glucose. Les expériences sont réalisées dans un sérum physiologique pour minimiser les artéfacts liés à des liaisons possibles avec des protéines. La réponse est mesurée après 30 min. d'incubation grâce à l'utilisation d'un 2-deoxyglucose marqué (2DeOG) et les résultats sont comparés à des inhibiteurs connus des transporteurs du glucose, c'est-à-dire la phlorétine et la génistéine. Les résultats sont exprimés graphiquement comme le pourcentage d'inhibition du transport du 2DeOG après 30 min. d'incubation par rapport au contrôle sans inhibiteur (figure 1).

L'extrait polyphénolique montre un effet d'inhibition du transport membranaire du glucose très important, quelquesoit les concentrations entre 5 et 500 µg / ml : on a une inhibition de l'absorption du 2DeOG de env. 30 % à une concentration de 5 µg / ml, inhibition qui passe à 80 % pour une concentration de 500 µg / ml. L'effet de l'extrait polyphénolique est au moins équivalent, voire supérieur, à celui de la génistéine ou de la phlorétine à des concentrations entre 1 et 100 µM, concentrations généralement reconnues pour leur efficacité vis-à-vis de l'effet recherché.

Cette expérience montre donc que la fraction polyphénolique riche en dihydrochalcones contribue à un contrôle de la charge en sucres par l'organisme, favorisant le contrôle de la surcharge pondérale, allant jusqu'à la prévention de certains diabètes.

### Exemple 2 : formulation d'une gélule à usage nutraceutique

Dans une gélule de 400 milligrammes, on incorpore 24 milligrammes de l'extrait polyphenolique selon l'invention. Les 376 milligrammes restants sont de la pectine de pomme.

La quantité d'extrait polyphénolique selon l'invention présente dans la gélule ainsi formulée correspond à l'apport de l'équivalent d'une pomme en dihydrochalcones.

### Exemple 3 : formulation d'un produit alimentaire nutraceutique

La masse blanche du yaourt contient de 2 à 7 % de sucres ajoutés et une teneur en matières grasses comprise entre 3 et 5 % de la masse brute du yaourt. Le pH de ce yaourt est compris entre 4,1 et 4,3.

On prépare un yaourt aux fruits qui contient de 8 à 10 % de fruits dans la masse blanche. L'extrait polyphénolique selon l'invention est directement incorporé dans la préparation de fruits (en même temps que les arômes et les colorants) destinée au yaourt. Néanmoins, comme les dihydrochalcones ne sont pas des molécules très solubles, 24 mg d'extrait polyphénolique peuvent être préalablement solubilisés dans 0.5 ml d'une solution hydroalcoolique avant leur incorporation dans la préparation de fruits destinée au yaourt ou directement dans le yaourt.

A ce dosage, l'extrait polyphénolique n'a pas d'impact organoleptique négatif. En outre, l'étiquetage peut revendiquer une formulation 100 % pomme si, en plus de l'utilisation de purées et/ou jus concentrés de pomme, l'aromatisation est réalisée à l'aide d'une essence de pomme et l'apport de sucre est réalisé à l'aide de sucre de pomme.

On obtient ainsi un produit laitier frais hypocalorique.

## Revendications

1. Utilisation, dans des préparations alimentaires ou nutraceutiques, d'une fraction polyphénolique riche en dihydrochalcones issue de fruits de la famille des *Rosaceae* dans le traitement cosmétique de mammifères afin de limiter la charge pondérale et améliorer l'aspect esthétique du corps.

2. Utilisation d'une fraction polyphénolique riche en dihydrochalcones selon la revendication 1, pour le traitement cosmétique de certaines formes d'obésité non pathologiques.

3. Utilisation selon l'une quelconque des revendications 1 ou 2 d'une fraction **caractérisée par le fait qu'**elle comprend au moins 10 % de polyphénols comprenant au moins 10 % en poids de phloridzine par rapport aux polyphénols totaux.

4. Utilisation selon l'une quelconque des revendications 1 à 3 d'une fraction **caractérisée par le fait qu'**elle contient de 10 à 70 % en poids de phloridzine par rapport aux polyphénols totaux.

5. Utilisation selon l'une quelconque des revendications 1 à 4 d'une fraction **caractérisée par le fait qu'**elle contient de l'acide chlorogénique.

6. Utilisation selon l'une quelconque des revendications 1 à 5 d'une fraction dont le rapport en poids de phloridzine par rapport au poids d'acide chlorogénique est supérieur ou égal à 1.

7. Utilisation selon l'une quelconque des revendications 1 à 6 d'une fraction **caractérisée par le fait qu'**elle contient de l'épicatéchine.

8. Utilisation selon l'une quelconque des revendications 1 à 7 d'une fraction dont le rapport en poids de phloridzine par rapport au poids d'épicatéchine est supérieur à 9.

9. Utilisation selon l'une quelconque des revendications 1 à 8 d'une fraction **caractérisée par le fait qu'**elle contient des quantités mineures de procyanidine B2, de quercitrine, de quercétine, d'acide p-coumarique et d'autres phénols.

10. Utilisation selon l'une quelconque des revendications 1 à 9 d'une fraction **caractérisée par le fait qu'**elle contient de l'acide cafféique dans des quantités telles que le rapport en poids de phloridzine par rapport au poids d'acide cafféique est supérieur à 4.

11. Utilisation selon l'une quelconque des revendications 1 à 10 d'une fraction dont la proportion en poids d'acide cafféique représente moins de 1 % des polyphénols totaux.

12. Utilisation selon l'une quelconque des revendications 1 à 11 d'une fraction **caractérisée par le fait qu'**elle contient de la phlorétine.

13. Utilisation selon l'une quelconque des revendications 1 à 12 d'une fraction **caractérisée par le fait que** les dihydrochalcones (phloridzine et phlorétine) sont présentes dans des proportions supérieures ou égales à 40 % en poids par rapport aux acides hydroxycinnamiques (acide cafféique, acide chlorogénique, acide p-coumarique).

14. Utilisation selon l'une quelconque des revendications 1 à 13 d'une fraction **caractérisée par le fait que** l'on procède à une extraction à partir de pommes matures.

15. Utilisation selon l'une quelconque des revendications 1 à 14 d'une fraction **caractérisée par le fait que** l'on procède à une extraction de la pomme *Malus sylvestris* Mill.

16. Utilisation d'une composition alimentaire ou nutraceutique comprenant une fraction polyphénolique telle que définie dans l'une quelconque des revendications 3 à 15 pour le traitement cosmétique afin de limiter la charge pondérale et améliorer l'aspect esthétique du corps.

17. Utilisation d'une composition alimentaire ou nutraceutique comprenant une fraction polyphénolique telle que définie dans l'une quelconque des revendications 3 à 15 pour le traitement cosmétique de certaines formes d'obésité non pathologiques.

## Claims

1. Use, in dietary or nutraceutical preparations, of a dihydrochalcone-rich polyphenolic fraction obtained from fruit of the Rosacea family in the cosmetic treatment of mammals in order to limit the weight and to improve the aesthetic appearance of the body.

2. Use of a dihydrochalcone-rich polyphenolic fraction according to Claim 1, for the cosmetic treatment of certain non-pathological forms of obesity.

3. Use according to either one of Claims 1 and 2 of a fraction, **characterized in that** it comprises at least 10% of polyphenols comprising at least 10% by weight of phloridzin relative to the total polyphenols.

4. Use according to any one of Claims 1 to 3 of a fraction, **characterized in that** it contains from 10% to 70% by weight of phloridzin relative to the total polyphenols.

5. Use according to any one of Claims 1 to 4 of a fraction, **characterized in that** it contains chlorogenic acid.

6. Use according to any one of Claims 1 to 5 of a fraction whose weight ratio of phloridzin relative to the weight of chlorogenic acid is greater than or equal to 1.

7. Use according to any one of Claims 1 to 6 of a fraction, **characterized in that** it contains epicatechin.

8. Use according to any one of Claims 1 to 7 of a fraction whose weight ratio of phloridzin relative to the weight of epicatechin is greater than 9.

9. Use according to any one of Claims 1 to 8 of a fraction, **characterized in that** it contains minor amounts of procyanidin B2, quercitrin, quercetin, p-coumaric acid and other phenols.

10. Use according to any one of Claims 1 to 9 of a fraction, **characterized in that** it contains caffeic acid in amounts such that the weight ratio of phloridzin relative to the weight of caffeic acid is greater than 4.

11. Use according to any one of Claims 1 to 10 of a fraction whose weight proportion of caffeic acid represents less than 1% of the total polyphenols.

12. Use according to any one of Claims 1 to 11 of a fraction, **characterized in that** it contains phloretin.

13. Use according to any one of Claims 1 to 12 of a fraction, **characterized in that** the dihydrochalcones (phloridzin and phloretin) are present in proportions of greater than or equal to 40% by weight relative to the hydroxycinnamic acids (caffeic acid, chlorogenic acid and p-coumaric acid).

14. Use according to any one of Claims 1 to 13 of a fraction, **characterized in that** an extraction is performed from ripe apples.

15. Use according to any one of Claims 1 to 14 of a fraction, **characterized in that** an extraction is performed on *Malus sylvestris* Mill apples.

16. Use of a dietary or nutraceutical composition comprising a polyphenolic fraction as defined in any one of Claims 3 to 15 for cosmetic treatment in order to limit the weight and to improve the aesthetic appearance of the body.

17. Use of a dietary or nutraceutical composition comprising a polypheholic fraction as defined in any one of Claims 3 to 15 for the cosmetic treatment of certain non-pathological forms of obesity.

## Patentansprüche

1. Verwendung einer Polyphenol-Fraktion mit einem hohen Gehalt an Dihydrochalkonen aus Früchten der Familie Rosaceae in Lebensmittelzubereitungen oder Zubereitungen von funktionellen Lebensmitteln zur kosmetischen Behandlung von Mammalia, um das Körpergewicht zu limitieren und das ästhetische Aussehen des Körpers zu verbessern.

2. Verwendung einer Polyphenol-Fraktion mit einem hohen Gehalt an Dihydrochalkonen nach Anspruch 1 für die kosmetische Behandlung einiger nicht pathologischer Formen von Adipositas.

3. Verwendung einer Fraktion nach einem der Ansprüche 1 oder 2, **dadurch gekennzeichnet, dass** sie mindestens 10 % Polyphenole mit mindestens 10 Gew.-% Phloridzin, bezogen auf die Polyphenole insgesamt, enthält.

4. Verwendung einer Fraktion nach einem der Ansprüche 1 bis 3, **dadurch gekennzeichnet, dass** sie mindestens 10 bis 70 Gew.-% Phloridzin, bezogen auf die Polyphenole insgesamt, enthält.

5. Verwendung einer Fraktion nach einem der Ansprüche 1 bis 4, **dadurch gekennzeichnet, dass** sie Chlorogensäure enthält.

6. Verwendung einer Fraktion nach einem der Ansprüche 1 bis 5, bei der das Verhältnis des Gewichts des Phloridzins und des Gewichts der Chlorogensäure größer oder gleich 1 ist.

7. Verwendung einer Fraktion nach einem der Ansprüche 1 bis 6, **dadurch gekennzeichnet, dass** sie Epicatechin enthält.

8. Verwendung einer Fraktion nach einem der Ansprüche 1 bis 7, bei der das Verhältnis des Gewichts des Phloridzins und des Gewichts des Epicatechins größer 9 ist.

9. Verwendung einer Fraktion nach einem der Ansprüche 1 bis 8, **dadurch gekennzeichnet, dass** sie kleinere Mengen von Procyanidin B2, Quercitrin, Quercetin, p-Cumarinsäure und weiteren Phenolen enthält.

10. Verwendung einer Fraktion nach einem der Ansprüche 1 bis 9, **dadurch gekennzeichnet, dass** sie Kaffeesäure in solchen Mengenanteilen enthält, dass das Verhältnis des Gewichts des Phloridzins und des Gewichts der Kaffeesäure größer 4 ist.

11. Verwendung einer Fraktion nach einem der Ansprüche 1 bis 10, **dadurch gekennzeichnet, dass** der Gewichtsanteil der Kaffeesäure weniger als 1 % der Polyphenole insgesamt ausmacht.

12. Verwendung einer Fraktion nach einem der Ansprüche 1 bis 11, **dadurch gekennzeichnet, dass** sie Phloretin enthält.

13. Verwendung einer Fraktion nach einem der Ansprüche 1 bis 12, **dadurch gekennzeichnet, dass** die Dihydrochalkone (Phloridzin und Phloretin) in Mengenanteilen von größer oder gleich 40 Gew.-%, bezogen auf die Hydroxyzimtsäuren (Kaffeesäure, Chlorogensäure, p-Cumarinsäure), vorliegen.

14. Verwendung einer Fraktion nach einem der Ansprüche 1 bis 13, **dadurch gekennzeichnet, dass** eine Extraktion ausgehend von reifen Äpfeln durchgeführt wird.

15. Verwendung einer Fraktion nach einem der Ansprüche 1 bis 14, **dadurch gekennzeichnet, dass** eine Extraktion des Apfels *Malus sylvestris* Mill durchgeführt wird.

16. Verwendung von Lebensmittelzusammensetzungen oder Zusammensetzungen von funktionellen Lebensmitteln, die eine Polyphenol-Fraktion enthalten, wie sie in einem der Ansprüche 3 bis 15 definiert ist, zur kosmetischen Behandlung, um das Körpergewicht zu limitieren und das ästhetische Aussehen des Körpers zu verbessern.

17. Verwendung von Lebensmittelzusammensetzungen oder Zusammensetzungen von funktionellen Lebensmitteln, die eine Polyphenol-Fraktion enthalten, wie sie in einem der Ansprüche 3 bis 15 definiert ist, zur kosmetischen Behandlung einiger nicht pathologischer Formen von Adipositas.
